# EUROPÄISCHE PATENTSCHRIFT

(11) **EP 4 197 995 B1**
(45) Veröffentlichungstag und Bekanntmachung des Hinweises auf die Patenterteilung: **09.07.2025**
(21) Anmeldenummer: 21215337.3
(22) Anmeldetag: 17.12.2021
(51) Int. Cl.: C07C 45/00, C07F 15/00

(54) **PT-XANTHEN-IOD-KOMPLEX UND PT-XANTHEN-BROM-KOMPLEX**
PT-XANTHENE-IODINE COMPLEX AND PT-XANTHENE BROMINE COMPLEX
COMPLEXE PT-XANTHÈNE-IODE ET COMPLEXE PT-XANTHÈNE-BROME

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- WO-A1-2017/191310
- WO-A2-2010/129030
- PETOCZ G ET AL: "Xantphos as cis- and trans-chelating ligand in square-planar platinum(II) complexes. Hydroformylation of styrene with platinum-xantphos-tin(II)chloride system", JOURNAL OF ORGANOMETALLIC CHEMISTRY, ELSEVIER, AMSTERDAM, NL, vol. 689, no. 7, 1 April 2004 (2004-04-01), pages 1188 - 1193, XP004496421, ISSN: 0022-328X, DOI: 10.1016/J.JORGANCHEM.2003.10.045
- VEEN VAN DER L A ET AL: "WIDE BITE ANGLE AMINE, ARSINE AND PHOSPHINE LIGANDS IN RHODIUM-AND PLATINUM/TIN-CATALYSED HYDROFORMYLATION", JOURNAL OF THE CHEMICAL SOCIETY, DALTON TRANSACTIONS, CHEMICAL SOCIETY. LETCHWORTH, GB, vol. 13, 1 January 2000 (2000-01-01), pages 2105 - 2112, XP001084858, ISSN: 1472-7773, DOI: 10.1039/B002126L

## Beschreibung

Die vorliegende Erfindung betrifft einen Pt-Xanthen-Iod-Komplex und Pt-Xanthen-Brom-Komplex, sowie deren Verwendung zur Katalyse einer Hydroformylierungsreaktion.

In C. Botteghi et al., Journal of Molecular Catalysis A: Chemical 200, (2003), 147-156 wird der Einsatz von Pt(Xantphos)Cl₂ zur Hydroformylierung von 2-Tosyloxystyrol beschrieben.

In WO 2010/129030 A2 wird ein Verfahren zur Herstellung von Vinylestern beschrieben. Hierbei wird eine Carbonsäure in Anwesenheit eines Katalysators mit Acetylen umgesetzt.

In Petöcz G. et al., "Xantphos as cis- and trans-chelating ligand in square-planar platinum(II) complexes. Hydroformylation of styrene with platinum-xantphos-tin(II)chloride system", Journal of Organometallic Chemistry 689(7):1188-1193, April 2004, wird ein Platin-Xantphos-Komplex beschrieben. Dieser wurde hergestellt und mittels NMR untersucht.

In van der Veen L.A. et al., "Wide bite angle amine, arsine and phosphine ligands in rhodiumand platinum/tin-catalysed hydroformylation", Journal of the Chemical Society, Dalton Transactions, Bd. 13, 1. Januar 2000, Seiten 2105-2112, wird ein Ligand-Pt(SnCl₃)CI-Komplex beschrieben. Dieser wird zur Hydroformylierung von 1-Octen eingesetzt.

Der vorliegende Erfindung lag die Aufgabe zugrunde, einen neuen Komplex bereitzustellen. Der Komplex soll hierbei bei der Katalyse von Hydroformylierungsreaktionen gegenüber dem im Stand der Technik beschriebenen Komplex Pt mit Cl₂ eine gesteigerte Ausbeute liefern.

Diese Aufgabe wird gelöst durch einen Komplex gemäß Anspruch 1.

Komplex umfassen:
a) Pt;
b) einen Liganden entsprechend der Formel (I): 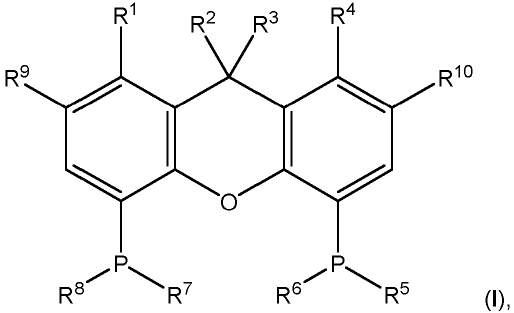
   wobei R¹, R², R³, R⁴, R⁵, R⁶, R⁷, R⁸ ausgewählt sind aus: -H, -(C₁-C₁₂)-Alkyl, -(C₆-C₂₀)-Aryl, und R⁹, R¹⁰ für -(C₁-C₁₂)-Alkyl stehen,
   und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) einen lod-Liganden oder Brom-Liganden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, iso-Butyl, sec-Butyl, tert-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform sind R², R³, R⁵, R⁶, R⁷, R⁸ ausgewählt aus: -(C₁-C₁₂)-Alkyl, -(C₆-C₂₀)-Aryl.

In einer Ausführungsform stehen R⁵, R⁶, R⁷, R⁸ für -(C₆-C₂₀)-Aryl.

In einer Ausführungsform stehen R⁵ und R⁶ für unterschiedliche Reste und R⁷ und R⁸ für unterschiedliche Reste.

In einer Ausführungsform stehen R² und R³ für -(C₁-C₁₂)-Alkyl.

In einer Ausführungsform R² und R³ für -CH₃.

In einer Ausführungsform stehen R¹ und R⁴ für -H.

In einer Ausführungsform stehen R⁹ und R¹⁰ für -^{t}Bu.

In einer Ausführungsform weist der Ligand entsprechend der Formel (I) die Struktur (1) auf:

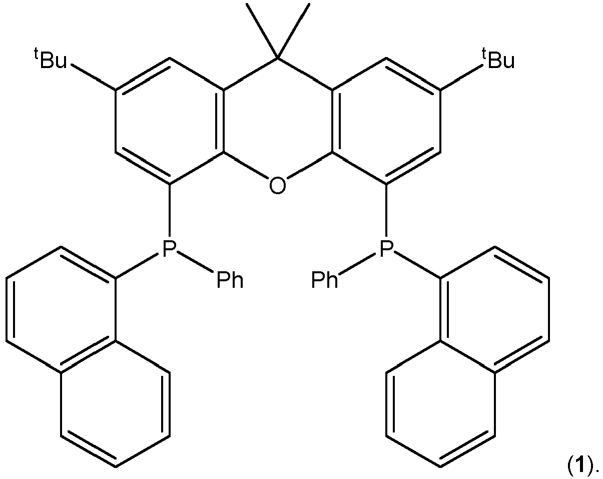

In einer Ausführungsform weist der Komplex genau einen Liganden entsprechend der Formel (I) auf.

In einer Ausführungsform weist der Komplex mindestens zwei lod-Liganden auf.

In einer Ausführungsform weist der Komplex genau zwei lod-Liganden auf.

In einer Ausführungsform weist der Komplex mindestens zwei Brom-Liganden auf.

In einer Ausführungsform weist der Komplex genau zwei Brom-Liganden auf.

Neben dem Komplex an sich, wird auch dessen Verwendung zur Katalyse einer Hydroformylierungsreaktion beansprucht.

Verwendung eines zuvor beschriebenen Komplexes zur Katalyse einer Hydroformylierungsreaktion.

Im Folgenden soll die Erfindung anhand von Ausführungsbeispielen näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und 1-Octen in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 18 h bei 80 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Variation des Halogens

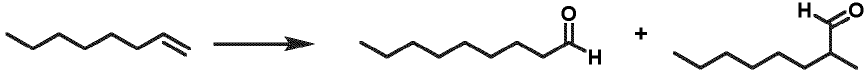

### Reaktionsbedingungen:

1.0 mmol 1-Octen, 0.5 mol% PtX₂, 2,0 Äquivalente Ligand (1), Lösungsmittel: Toluol, p(CO/H₂): 40 bar, T: 80 °C, t: 18 h.

### Ausbeuten:

| Ligand | Halogen | Ausbeute [%] |
|---|---|---|
| 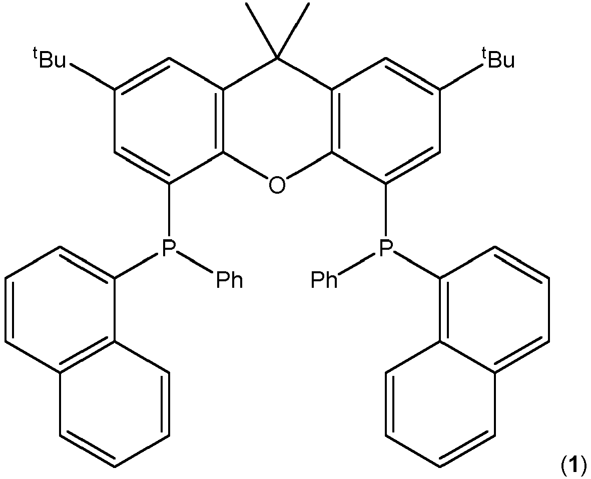 | I / Br / Cl | 96/24/0 |

Wie die Versuchsergebnisse zeigen, wird die Aufgabe durch den erfindungsgemäßen Komplex gelöst.

## Patentansprüche

1. Komplex umfassen:
a) Pt;
b) einen Liganden entsprechend der Formel (I): 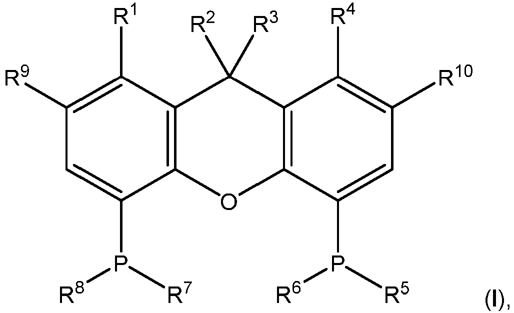
wobei R¹, R², R³, R⁴, R⁵, R⁶, R⁷, R⁸ ausgewählt sind aus: -H, -(C₁-C₁₂)-Alkyl, -(C₆-C₂₀)-Aryl, und R⁹, R¹⁰ für -(C₁-C₁₂)-Alkyl stehen,
und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) einen lod-Liganden oder Brom-Liganden.

2. Komplex nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Komplex nach einem der Ansprüche 1 oder 2,
wobei R⁵, R⁶, R⁷, R⁸ für -(C₆-C₂₀)-Aryl stehen.

4. Komplex nach einem der Ansprüche 1 bis 3,
wobei R² und R³ für -(C₁-C₁₂)-Alkyl stehen.

5. Komplex nach einem der Ansprüche 1 bis 4,
wobei R¹ und R⁴ für -H stehen.

6. Komplex nach einem der Ansprüche 1 bis 5,
wobei der Ligand entsprechend der Formel (I) die Struktur (1) aufweist:

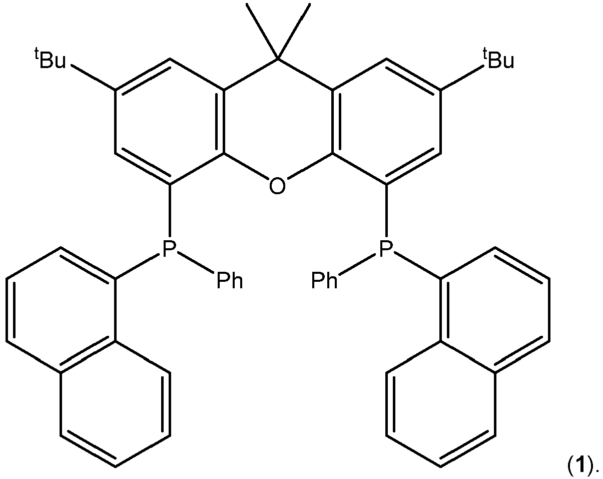

7. Komplex nach einem der Ansprüche 1 bis 6,
wobei der Komplex genau einen Liganden entsprechend der Formel (I) aufweist.

8. Komplex nach einem der Ansprüche 1 bis 7,
wobei der Komplex mindestens zwei lod-Liganden aufweist.

9. Komplex nach Anspruch 8,
wobei der Komplex genau zwei lod-Liganden aufweist.

10. Komplex nach einem der Ansprüche 1 bis 7,
wobei der Komplex mindestens zwei Brom-Liganden aufweist.

11. Komplex nach Anspruch 10,
wobei der Komplex genau zwei Brom-Liganden aufweist.

12. Verwendung eines Komplexes nach einem der Ansprüche 1 bis 11 zur Katalyse einer Hydroformylierungsreaktion.

## Claims

1. Complex comprising:
a) Pt;
b) a ligand conforming to the formula (I): 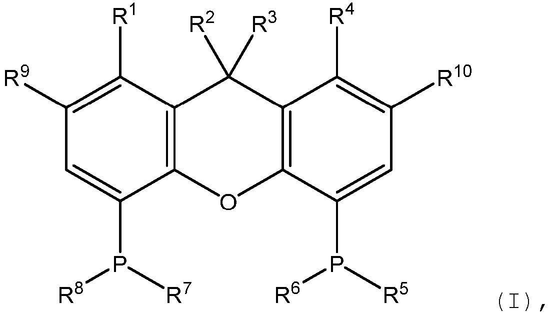
where R¹, R², R³, R⁴, R⁵, R⁶, R⁷, R⁸ are selected from: -H, -(C₁-C₁₂)-alkyl, -(C₆-C₂₀)-aryl, and
R⁹, R¹⁰ are -(C₁-C₁₂)-alkyl,
and, if R¹, R², R³, R⁴, R⁵, R⁶, R⁷, R⁸ are -(C₆-C₂₀)-aryl, the aryl ring may have substituents selected from: -(C₁-C₁₂)-alkyl, -O-(C₁-C₁₂)-alkyl;
c) an iodine ligand or bromine ligand.

2. Complex according to Claim 1,
where R², R³, R⁵, R⁶, R⁷, R⁸ are selected from: -(C₁-C₁₂)-alkyl, -(C₆-C₂₀) -aryl.

3. Complex according to either of Claims 1 and 2,
where R⁵, R⁶, R⁷, R⁸ are -(C₆-C₂₀) -aryl.

4. Complex according to any of Claims 1 to 3,
where R² and R³ are -(C₁-C₁₂)-alkyl.

5. Complex according to any of Claims 1 to 4,
where R¹ and R⁴ are -H.

6. Complex according to any of Claims 1 to 5,
wherein the ligand conforming to the formula (I) has the structure (1):

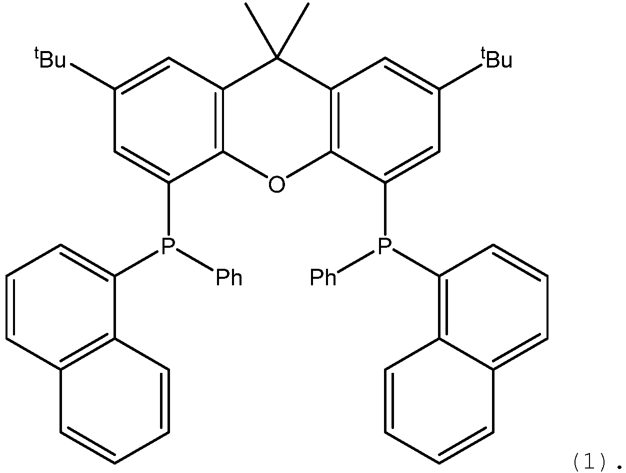

7. Complex according to any of Claims 1 to 6,
wherein the complex has exactly one ligand conforming to the formula (I).

8. Complex according to any of Claims 1 to 7,
wherein the complex has at least two iodine ligands.

9. Complex according to Claim 8,
wherein the complex has exactly two iodine ligands.

10. Complex according to any of Claims 1 to 7,
wherein the complex has at least two bromine ligands.

11. Complex according to Claim 10,
wherein the complex has exactly two bromine ligands.

12. Use of a complex according to any of Claims 1 to 11 for catalysis of a hydroformylation reaction.

## Revendications

1. Complexe comprenant :
a) du Pt ;
b) un ligand selon la formule (I) : 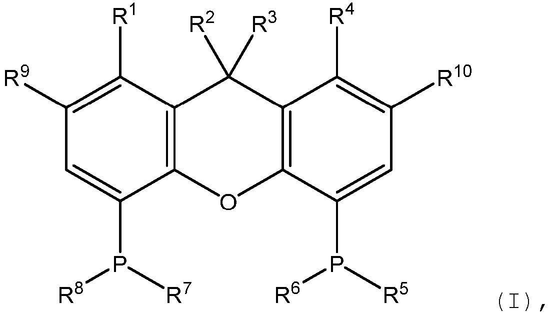
R¹, R², R³, R⁴, R⁵, R⁶, R⁷, R⁸ étant choisis parmi : -H, - (C₁-C₁₂)-alkyle, - (C₆-C₂₀) -aryle et
R⁹, R¹⁰ représentant - (C₁-C₁₂)-alkyle,
et pour le cas où R¹, R², R³, R⁴, R⁵, R⁶, R⁷, R⁸ représentent - (C₆-C₂₀) -aryle, le cycle aryle pouvant présenter des substituants qui sont choisis parmi : -(C₁-C₁₂)-alkyle, - O-(C₁-C₁₂)-alkyle ;
c) un ligand iode ou un ligand brome.

2. Complexe selon la revendication 1,
R², R³, R⁵, R⁶, R⁷, R⁸ étant choisis parmi : -(C₁-C₁₂)-alkyle, - (C₆-C₂₀) -aryle.

3. Complexe selon l'une des revendications 1 ou 2,
R⁵, R⁶, R⁷, R⁸ représentant -(C₆-C₂₀) -aryle.

4. Complexe selon l'une des revendications 1 à 3,
R² et R³ représentant -(C₁-C₁₂)-alkyle.

5. Complexe selon l'une des revendications 1 à 4,
R¹ et R⁴ représentant -H.

6. Complexe selon l'une des revendications 1 à 5,
le ligand selon la formule (I) présentant la structure (1) :

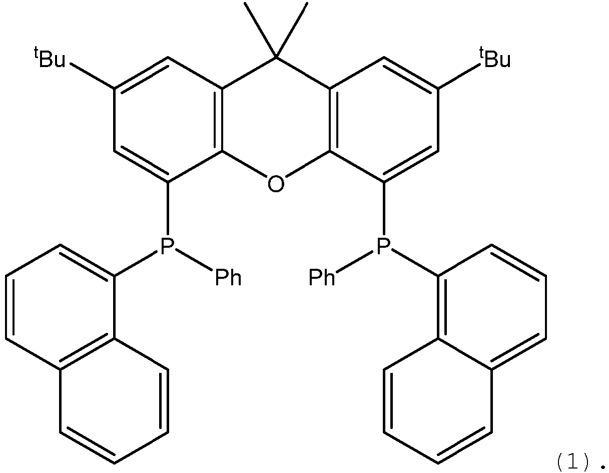

7. Complexe selon l'une des revendications 1 à 6,
le complexe présentant exactement un ligand selon la formule (I).

8. Complexe selon l'une des revendications 1 à 7,
le complexe présentant au moins deux ligands iode.

9. Complexe selon la revendication 8,
le complexe présentant exactement deux ligands iode.

10. Complexe selon l'une des revendications 1 à 7,
le complexe présentant au moins deux ligands brome.

11. Complexe selon la revendication 10,
le complexe présentant exactement deux ligands brome.

12. Utilisation d'un complexe selon l'une des revendications 1 à 11 pour la catalyse d'une réaction d'hydroformylation.
